# DEMANDE DE BREVET EUROPEEN

(11) **EP 4 276 853 A1**
(43) Date de publication de la demande: **15.11.2023**
(21) Numéro de dépôt: 23171908.9
(22) Date de dépôt: 05.05.2023
(51) Int. Cl.: G16H 70/40, G16H 20/10

(54) **PROCÉDÉ D'ASSISTANCE DANS UNE DÉMARCHE DE FORMULATION D'UNE COMPOSITION**

(30) Priorité: 09.05.2022 FR 2204356
(71) Demandeur: Activ'Inside, 33750 Beychac-et-Caillau (FR)
(72) Inventeur: GAUDOUT, David, 33360 CARIGNAN DE BORDEAUX (FR); REY, Stéphane, 26200 MONTELIMAR (FR); LEMAIRE, Benoît, 33500 LIBOURNE (FR); FROIDEVAL, Victoria, 33290 LE PIAN MEDOC (FR)
(74) Mandataire: Aquinov

(57) **Abrégé**

La présente invention concerne un procédé d'assistance dans la formulation d'une composition mis en oeuvre par un terminal mobile comprenant : l'affichage sur l'écran du terminal mobile d'une interface de sélection, par un utilisateur, d'un ingrédient ; sélection d'au moins une plage de doses de référence associée audit ingrédient sélectionné ; affichage sur l'écran du terminal mobile d'une interface de sélection d'une dose de l'ingrédient sélectionné, comprenant un élément graphique de sélection manipulable ; détermination de la dose de l'ingrédient à partir de l'élément graphique de sélection ; génération et affichage d'au moins une information visuelle sur l'écran du terminal, en fonction de la dose déterminée à l'étape précédente et de l'au moins une plage de doses de référence sélectionnée.

## Description

### Domaine technique

L'invention se rapporte au domaine technique des activités nutraceutiques. Plus précisément, l'invention a pour objet un procédé d'assistance dans la formulation d'une composition, notamment nutraceutique, mis en oeuvre par un terminal mobile.

### Etat de l'art

Dans ce domaine, il est connu de faire appel à des façonniers, c'est-à-dire des entreprises spécialisées dans la conception, formulation, préparation de composition y compris le choix de la forme galénique, par exemple nutraceutique (sous forme de comprimés, gélules, gummies, sachets etc.) ou cosmétique (sous forme de crèmes, lotions etc.), à partir d'ingrédients actifs eux-mêmes développés par des entreprises spécialisées dans le développement de tels ingrédients. Il est également possible de faire appel à des sociétés de conseils spécialisés dans la formulation des ingrédients, du choix de la forme galénique et dans la réglementation associée.

Dans le cadre de leurs activités commerciales et marketing, ces différentes entreprises font appel à des commerciaux ou des chefs produit spécialisés dans le domaine pour organiser et rencontrer des clients ou des prospects de l'entreprise, notamment des laboratoires de compléments alimentaires ou des façonniers par exemple, afin de leur proposer de nouvelles compositions intégrant des ingrédients et permettant de répondre à leurs besoins ainsi qu'aux besoins des consommateurs. Par exemple, lorsqu'une entreprise a développé de nouveaux ingrédients, lorsqu'elle est à la recherche de nouveaux marchés ou de nouveaux clients, ou encore lorsque l'entreprise est confrontée à de nouvelles problématiques réglementaires nécessitant d'adapter leur offre.

Aussi, le commercial ou le chef produit peut, par exemple, être amené à rencontrer des clients lors d'évènements, par exemple des salons internationaux, au cours desquels il doit présenter dans un intervalle de temps réduit l'ensemble des produits et services permettant de répondre aux besoins du client.

Toutefois, lors de sa rencontre avec le client, le commercial ou le chef produit ne dispose très souvent de peu d'informations, quant aux besoins du client. En outre, dans ce domaine, le commercial doit, certes, connaître les caractéristiques, performances et prix du produit, mais également l'environnement réglementaire associé à l'ingrédient actif dépendant en sus des territoires de commercialisation envisagés par le client. Par environnement réglementaire, on entend notamment l'ensemble des connaissances telles que la disponibilité d'allégations de santé, les doses réglementaires, les incompatibilités connues avec d'autres ingrédients, les éventuels risques de requalification en médicament, et ce pour l'ensemble des territoires dans lesquels le produit final est susceptible d'être commercialisé. Certes, le commercial dans ce domaine, est pluridisciplinaire et exige des connaissances approfondies, néanmoins, les besoins du client peuvent être multiples et relever d'une discipline ou nécessiter des connaissances spécifiques. Aussi, le commercial ou le chef produit nécessite des connaissances scientifiques approfondies, en particulier de connaitre le contexte réglementaire associé à chaque ingrédient dans le domaine des produits nutritionnels, des compléments alimentaires, cosmétiques, voire des médicaments. En effet, la connaissance de la réglementation en vigueur dans les territoires d'intérêt est indispensable afin de déterminer la dose applicable, les éventuelles incompatibilités réglementaires associées à ladite dose déterminée, ou les risques de requalification en médicament associées à ladite dose déterminée.

Ceci implique que le commercial ou le chef produit, malgré son expérience multidisciplinaire, peut avoir besoin d'une ou plusieurs expertises spécifiques afin de mener à bien l'évaluation et la compréhension des besoins du client afin de lui proposer une offre commerciale, soit une composition comprenant au moins un ingrédient ainsi que la dose associée audit ingrédient. En outre, le peu ou l'absence d'informations dont il dispose peut induire un biais dans l'émission de l'offre commerciale adaptée aux besoins du client dans un intervalle de temps court et de procéder à une analyse multifactorielle et pluridisciplinaire. Ces points sont particulièrement critiques dans un environnement réglementaire complexe, dans la mesure où l'absence d'information peut l'empêcher de présente une offre, ou causer une erreur ou un délai de traitement important susceptible d'entraîner des dommages souvent économiques importants pour l'entreprise, par exemple la perte de clients ou de nouveaux marchés.

Une solution pourrait consister à organiser une rencontre conjointe du commercial ou du chef produit avec des experts scientifiques, technologiques et/ou réglementaires. Toutefois, il n'est pas toujours possible d'organiser une telle rencontre, dans un temps acceptable, avec au moins un expert disposant de ces compétences requises, d'une part du fait du nombre croissant de rencontre effectuée par le commercial ou le chef produit au regard du nombre d'experts disponibles et d'autre part du fait des coûts économiques qu'un tel déplacement engendrerait.

### Résumé de l'invention

Il existe ainsi un besoin pour une solution permettant à un commercial ou un chef produit, de réaliser une rencontre en autonomie avec un client dans une démarche de fourniture d'une offre commerciale complète d'une composition comprenant au moins un ingrédient associé à une dose spécifique, considérant l'ensemble des besoins du client, qu'ils soient économiques, réglementaires, et ce en fonction des territoires de commercialisation, et lui permettant d'avoir accès à une expertise spécifique afin qu'une offre commerciale puisse être émise le plus rapidement possible et de façon la plus fiable possible.

La présente invention se place dans ce contexte et vise à répondre à ce besoin.

A ces fins, l'invention a pour objet un procédé d'assistance dans la formulation d'une composition mis en oeuvre par un terminal mobile, dans lequel le procédé comprend au moins les étapes suivantes :
a. Affichage sur l'écran du terminal mobile d'une interface de sélection, par un utilisateur du terminal mobile, d'un ingrédient parmi une pluralité d'ingrédients prédéterminés stockés dans une base de données ;
b. Sélection d'au moins une plage de doses de référence associée audit ingrédient sélectionné parmi une pluralité de doses de référence stockées dans une base de données,
c. Affichage sur l'écran du terminal mobile d'une interface de sélection d'une dose de l'ingrédient sélectionné, comprenant un élément graphique de sélection manipulable par un utilisateur du terminal mobile,
d. Détermination de la dose de l'ingrédient parmi une pluralité de doses prédéterminées dudit ingrédient, à partir de l'élément graphique de sélection,
e. Génération et affichage d'au moins une information visuelle sur l'écran du terminal, en fonction de la dose déterminée à l'étape précédente et de l'au moins une plage de doses de référence sélectionnée.

Grâce à l'invention, un utilisateur, par exemple un commercial ou un chef produit, équipé du terminal mobile capable de mettre en oeuvre le procédé selon l'invention, peut organiser, en autonomie, une rencontre avec des clients et lui permet d'accéder à toute l'expertise nécessaire pour proposer une offre commerciale rapide, fiable et prenant en compte les besoins du client, en respectant les contraintes notamment réglementaires. En outre, le procédé mis en oeuvre par un terminal mobile permet de suivre une démarche précise, notamment selon différents besoins, chacun adapté à une situation donnée, afin de faire évoluer, d'affiner, de préciser l'offre commerciale en fonction des besoins hiérarchisés du client. Aussi, le procédé nécessite l'implémentation des besoins du client en amont ou lors de la mise en oeuvre du procédé selon l'invention, pouvant prendre la forme de questions, de sélection de filtres, ou encore d'options prédéterminés.

Ces questions, filtres, options, remplis par l'utilisateur du terminal mobile, permettent ainsi de guider et de faciliter le recueil des besoins du client, afin d'identifier les points clés lors de la rencontre.

A l'issue de la rencontre et du recueil des besoins du client, le procédé selon l'invention permet à l'utilisateur de conclure la rencontre par la formulation d'une ou d'une pluralité d'offre commerciale qu'il établit en fonction des besoins du client, de la réglementation en vigueur et de la faisabilité de la composition, objet de l'offre commerciale. Bien évidemment, cette offre pourra être enrichie, par l'utilisateur après la rencontre.

Selon l'invention, on entend par terminal mobile un ordinateur portable, un dispositif mobile, un téléphone intelligent ou une tablette électronique. Ce terminal mobile comprend divers composants matériels, dont un ou plusieurs processeurs à un ou plusieurs coeurs et une mémoire.

Le cas échéant, le contrôleur dudit terminal mobile pourra comporter tout ou partie desdits composants matériels, ainsi que des composants logiciels. De préférence, le contrôleur dudit terminal mobile pourra comporter une application logicielle, par exemple téléchargée dans une mémoire du terminal mobile apte à contrôler les différents éléments du terminal mobile, notamment l'écran du terminal mobile, par exemple en fonction des données saisies par l'intermédiaire de l'interface ; et mettre en oeuvre le procédé selon l'invention. En variante, ladite application logicielle pourra être partiellement téléchargée dans une mémoire du terminal mobile et hébergée partiellement sur un serveur distant. Dans d'autres cas, l'application logicielle pourra être partiellement ou totalement virtualisé par le biais d'une architecture en nuage.

On entend par interface du terminal mobile une interface de saisie de données, par exemple une interface tactile, une interface vocale ou une interface gestuelle.

Avantageusement, le terminal mobile est capable d'accéder à une base de données distante dans laquelle sont stockées une pluralité de données prédéterminées. A titre d'exemple, la base de données peut comprendre une liste d'ingrédients prédéterminées, y compris des données scientifiques, réglementaires, économiques relatives à chaque ingrédient. Ainsi, la base de données comprend une pluralité de doses de référence associée auxdits ingrédients stockés dans la base de données.

Avantageusement, la base de données comprend des données associées à chaque ingrédient telles qu'une thématique santé, par exemple le stress, les performances cognitives etc., une allégation de santé, c'est-à-dire des propriétés sanitaires et/ou nutritionnelles de l'ingrédient, par exemple un lien entre l'ingrédient et un bénéfice sur l'état de santé d'un consommateur, par un exemple un extrait de safran ayant un bénéfice sur le stress ; une preuve d'efficacité de l'ingrédient, par exemple des études cliniques publiées dans des revues spécialisées, ou des utilisations traditionnelles ou populaires de l'ingrédient, des territoires de commercialisation, par exemple en Europe, Etats-Unis, Corée du Sud etc., au moins une certification qualité de l'ingrédient, par exemple une certification biologique, kasher, halal, végan etc; mais également au moins une forme galénique, un critère prenant en compte le nombre d'unité de remplissage, par exemple en fonction de la forme galénique d'intérêt, ou au moins une forme de packaging.

Aussi, pour faciliter la démarche de recueil des besoins du client en vue de proposer une offre commerciale satisfaisante, l'utilisateur peut avantageusement sélectionner un ou plusieurs critères prédéterminés et stockés dans la base de données de tel sorte que l'interface de sélection affichée sur l'écran du terminal mobile par l'utilisateur affiche un ingrédient parmi une pluralité d'ingrédients, ladite pluralité d'ingrédients déterminé étant associée à la sélection d'au moins un des critères précédemment sélectionnés par l'utilisateur, et ledit ingrédient est associé à une plage de doses de référence. Avantageusement, la plage de doses de référence est une pluralité de sous-plage de doses de référence.

La sélection d'au moins un de ces critères permet ainsi de déterminer les ingrédients actifs les plus pertinents permettant de répondre aux besoins du client en fonction des critères précédemment sélectionnés. Préférentiellement, l'ingrédient associé à la plage de doses de référence est sélectionné en fonction d'au moins un critère choisi parmi la sélection d'une allégation de santé, d'une preuve d'efficacité, d'un territoire de commercialisation, d'une valeur de référence, d'une forme galénique, et leur combinaison.

Avantageusement, la preuve d'efficacité est choisie parmi une preuve scientifique, traditionnelle et populaire. La preuve scientifique peut être issue de données scientifique issues de bases de données publiques ou issues de base de données propriétaires, lesdites données étant prédéterminées et stockées dans la base de données. Par exemple, les preuves scientifiques démontrant l'efficacité d'un ingrédient associé à une plage de doses, peuvent être issues d'articles scientifiques ou d'études cliniques disponibles, par exemple via le site PubMed.

La sélection par l'utilisateur de l'application logicielle, d'au moins un de ces critères est optionnelle et vise uniquement à faciliter la démarche de recueil des besoins du client. Il en résulte, l'affichage, sur l'écran du terminal mobile d'une interface de sélection, par l'utilisateur du terminal mobile, d'un ingrédient parmi une pluralité d'ingrédients prédéterminés stockés dans une base de données, ladite pluralité d'ingrédients prédéterminés correspondant à la totalité des ingrédients prédéterminés stockée dans la base de données.

Dès lors, l'utilisateur peut sélectionner au moins une plage de doses de référence associée audit ingrédient sélectionné parmi la pluralité de doses de référence stockées dans la base de données. Une fois sélectionné, l'écran du terminal mobile peut afficher une nouvelle interface, par exemple sous forme d'une nouvelle page, d'un nouvel onglet, d'une fenêtre pop-up ou pop-in, dites interface de sélection d'une dose de l'ingrédient sélectionné, ladite interface de sélection comprenant au moins un élément graphique de sélection manipulable par l'utilisateur du terminal mobile.

De préférence, l'élément graphique de sélection manipulable est un élément graphique de sélection déplaçable dans un plan cartésien selon un axe, un repère ou une position angulaire, par exemple un curseur déplaçable selon un axe horizontal ou vertical.

Selon une variante, l'élément graphique de sélection manipulable est un élément graphique de sélection modifiable selon une forme et/ou une taille et/ou une couleur, par exemple un dégradé de couleur correspondant à certaines valeurs quantifiables.

Selon une autre variante, l'élément graphique de sélection manipulable est un élément graphique de saisie, préférentiellement un composant de saisie d'une réponse quantifiable, par exemple un nombre, via l'interface. A titre d'exemple, la saisie de la réponse quantifiable permet, via l'interface, l'affichage d'une information visuelle indiquant la dose de l'ingrédient sélectionné, l'information visuelle peut-être l'élément graphique de sélection de la dose de l'ingrédient.

Ainsi, de préférence, l'élément graphique de sélection est déplaçable dans un plan cartésien selon un axe, un repère ou une position angulaire ; et/ou l'élément graphique de sélection est modifiable selon une forme et/ou une couleur.

Avantageusement, l'élément graphique de sélection est modifié lors de la sélection de la dose par l'utilisateur du terminal mobile, par exemple, lors du déplacement du curseur selon un axe horizontal, ou du changement d'une forme donnée, de son agrandissement ou de son éventuelle modification colorimétrique.

Une fois la dose sélectionnée par l'utilisateur du terminal mobile, la dose de l'ingrédient est déterminée parmi la pluralité de doses prédéterminées dudit ingrédient, à partir de l'élément graphique de sélection. Dès lors, au moins une information visuelle est générée et affichée sur l'écran du terminal, en fonction de la dose déterminée précédemment et de l'au moins une plage de doses de référence sélectionnée.

De préférence, l'information visuelle est générée et affichée en fonction de la comparaison entre la dose déterminée et la plage de doses de référence.

Avantageusement, l'affichage de l'information visuelle est mis en oeuvre par le changement de couleur de ladite information visuelle ou de l'affichage de l'information visuelle elle-même. Par exemple, l'information visuelle peut être un logo et/ou un texte prédéterminé, celle-ci peut-être par exemple grisé et affiché dans une autre couleur en fonction de la dose déterminée précédemment, en particulier de la comparaison avec la plage de doses de référence.

L'information visuelle peut être l'affichage de la présence d'au moins une allégation de santé en fonction dudit ingrédient sélectionné et de la dose déterminée, et/ou l'affichage d'au moins une incompatibilité réglementaire, et/ou d'au moins un risque de requalification en médicament, et/ou d'au moins un défaut d'autorisation réglementaire et/ou d'au moins une forme galénique, et/ou d'au moins le nombre d'unité de remplissage et/ou d'au moins la forme du packaging final.

Avantageusement, l'interface de sélection de l'au moins un ingrédient comporte au moins un premier bloc comprenant un filtre et ledit au moins un ingrédient est sélectionné parmi la pluralité d'ingrédients stockée dans la base de données, en fonction d'au moins une réponse audit bloc.

Par exemple, la base de données comprend une pluralité de données complémentaires en sus de la pluralité d'ingrédients prédéterminés, au moins une donnée complémentaire étant associée à chaque ingrédient de ladite pluralité d'ingrédients prédéterminés. Ces autres données telles que les données relatives à une thématique santé, à une allégation de santé, à une preuve d'efficacité, à un territoire de commercialisation, à une certification qualité. Le cas échéant, l'étape de sélection comporte la recherche, dans la base de données, d'au moins un ingrédient, voire de l'unique ingrédient, dans la base de données, dont ladite au moins une donnée complémentaire associée correspond à une réponse de l'utilisateur entrée via le filtre du premier bloc.

De préférence, le premier bloc de filtres comprend une interface de sélection d'une thématique santé, et/ou d'au moins un territoire de commercialisation, et/ou d'un volume de composition.

Par exemple, la sélection de la thématique santé parmi la pluralité de thématique santé prédéterminé et stocké dans la base de données permet de réduire la liste d'ingrédients disponibles parmi la pluralité d'ingrédient stocké dans la base de données à la suite de la réponse de l'utilisateur entrée via l'interface de sélection de la thématique santé. A titre d'exemple, l'utilisateur peut sélectionner une thématique santé englobant les troubles liés au stress, excluant ainsi les ingrédients n'ayant aucune efficacité dans ladite thématique sélectionnée.

Plus préférentiellement, le premier bloc de filtres comprend une interface de sélection d'une thématique santé, et d'au moins un territoire de commercialisation, encore plus préférentiellement, le premier bloc de filtres comprend une interface de sélection d'une thématique santé, et d'au moins un territoire de commercialisation et d'un volume de composition.

De préférence, chaque filtre de l'interface de sélection peut prendre la forme d'une liste déroulante comprenant une pluralité d'item.

De préférence, l'interface de sélection de l'au moins un ingrédient comporte au moins un deuxième bloc comprenant un filtre et ledit au moins un ingrédient est sélectionné parmi la pluralité d'ingrédients stockée dans la base de données, en fonction d'au moins une réponse audit bloc.

Par exemple, la base de données comprend une pluralité de données complémentaires en sus de la pluralité d'ingrédients prédéterminés, au moins une donnée complémentaire étant associée à chaque ingrédient de ladite pluralité d'ingrédients prédéterminés. Ces autres données peuvent être des données relatives à une allégation de santé et/ou d'au moins une certification qualité. Ainsi, de préférence, ledit 2^{ème} bloc de filtre comprend une interface de sélection d'au moins une allégation de santé et/ou d'au moins une certification qualité. Avantageusement, la certification qualité comprend les données complémentaires suivantes : ingrédient biologique, naturel, halal, kasher, vegan et leur combinaison.

Selon une variante, le premier et le deuxième bloc de filtres peuvent constituer un seul bloc de filtre. Aussi, l'interface de sélection de l'au moins un ingrédient comporte un bloc comprenant au moins un filtre et ledit au moins un ingrédient est sélectionné parmi la pluralité d'ingrédients stockée dans la base de données, en fonction d'au moins une réponse audit bloc

Selon un objet préféré, le procédé comprend également une étape d'affichage sur l'écran du terminal mobile d'une interface de présentation de chaque ingrédient. Avantageusement, l'interface de présentation de l'ingrédient permet à l'utilisateur de consulter une description associée audit ingrédient, les certifications qualité associées audit ingrédient, les preuves d'efficacité associées audit ingrédient, les allégations de santé associées audit ingrédient, et également une matrice de doses regroupant la pluralité des plages de doses prédéterminée associée audit ingrédient.

Avantageusement, dès lors l'ingrédient est sélectionné et sa dose déterminée, le procédé comprend également une étape de sélection d'au moins un 2^{ème} ingrédient, dit ingrédient suggéré, parmi la pluralité d'ingrédients stockée dans une base de données en fonction de l'ingrédient sélectionné et de la dose dudit ingrédient déterminée et une étape d'affichage dudit ingrédient suggéré sur l'écran du terminal. Cette étape de sélection vise à compléter la composition avec au moins un ingrédient supplémentaire. Celui-ci peut-être un ingrédient actif, par exemple un extrait de safran ; ou non actif, par exemple un excipient. Aussi, à partir de la pluralité de données stockée dans la base de données associé audit ingrédient, un 2^{ème} ingrédient peut être sélectionné en fonction desdits données associées au 1^{er} ingrédient sélectionné.

De préférence, la sélection de l'ingrédient suggéré est mise en oeuvre en fonction d'au moins un critère de compatibilité et/ou d'interaction entre les ingrédients sélectionnés, stockés dans la base de données. A titre d'exemple, les critères de compatibilité et/ou d'interaction peuvent être des critères d'efficacité, notamment un effet synergique connu entre les ingrédients sélectionnés. Selon une variante, cela peut-être un critère marketing, par exemple un ingrédient naturel, biologique.

De préférence, le procédé selon l'invention comprend en outre, une étape de génération et d'affichage d'au moins une information économique de la composition sur l'écran du terminal, en fonction du volume de composition sélectionné. Par exemple, un prix de vente indicatif en fonction du volume de composition sélectionné.

De préférence, le procédé comprend également la génération et l'affichage d'une nouvelle interface de sélection, par l'utilisateur du terminal mobile, d'au moins une option de service parmi une pluralité d'option de services prédéterminés stockés dans la base de données. La sélection d'au moins une option de services permettant de moduler l'information économique de la composition sur l'écran du terminal.

Avantageusement, l'affichage d'au moins une information économique modifiée de la composition est mise en oeuvre au moyen d'une synchronisation instantanée, sur l'écran du terminal, en fonction de l'au moins une option de service sélectionnée.

La présente invention a également pour objet un programme d'ordinateur comprenant un code de programme contenant des instructions qui, lorsqu'elles sont exécutées par un processeur, sont conçues pour mettre en oeuvre les étapes du procédé selon l'invention.

Enfin, l'invention a également pour autre objet, un support de stockage de données sur lequel est enregistré le programme d'ordinateur selon l'invention.

La présente invention est maintenant décrite à l'aide d'exemples uniquement illustratifs et nullement limitatifs de la portée de l'invention, et à partir des dessins annexés, dessins sur lesquels les différentes figures représentent :
[Fig. 1] représente, partiellement et schématiquement, un procédé d'assistance dans la formulation d'une composition mis en oeuvre par le terminal mobile.
[Fig. 2] représente, partiellement et schématiquement, une vue de l'écran du terminal mobile lors d'une étape du procédé de la [Fig. 1] ;
[Fig. 3] représente, partiellement et schématiquement, une vue de l'écran du terminal mobile lors d'une autre étape du procédé de la [Fig. 1] ;
[Fig. 4] représente, partiellement et schématiquement, une vue de l'écran du terminal mobile lors d'une autre étape du procédé de la [Fig. 1] ;
[Fig. 5] représente, partiellement et schématiquement, une vue de l'écran du terminal mobile lors d'une autre étape du procédé de la [Fig. 1] ; et
[Fig. 6] représente, partiellement et schématiquement, une vue de l'écran du terminal mobile lors d'une autre étape du procédé de la [Fig. 1].

Dans la description qui suit, les éléments identiques, par structure ou par fonction, apparaissant sur différentes figures conservent, sauf précision contraire, les mêmes références.

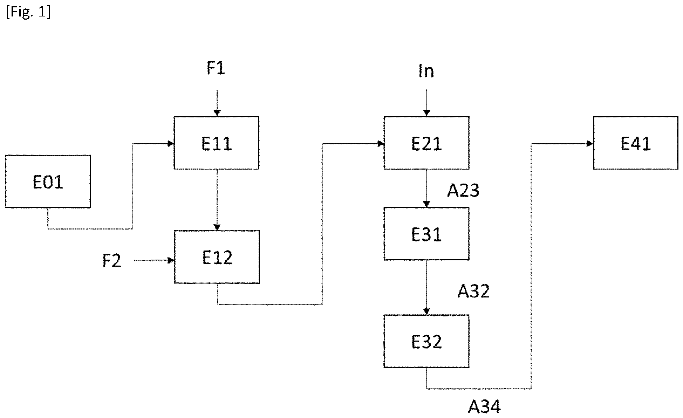

On va désormais décrire un exemple de procédé mis en oeuvre par l'application logicielle A lors d'une rencontre réalisée entre l'utilisateur, par exemple un commercial ou un chef produit et le client [Fig. 1], l'application logicielle contrôlant l'écran 11, par exemple d'une tablette 1, notamment en fonction des interactions de l'utilisateur, avec la tablette.

Au début de la rencontre avec le client, l'utilisateur de la tablette peut initialiser le démarrage de l'application A, permettant l'affichage d'une interface de création d'un nouveau projet affichée sur l'écran 11 permettant ainsi la création d'une nouvelle formulation ou d'une pluralité de formulation, c'est-à-dire d'une composition nutritionnelle, nutraceutique, nutricosmétique, voire cosmétique.

Aussi, dans une étape E01, l'utilisateur peut initialiser l'affichage de l'interface de création d'une nouvelle composition affichée sur l'écran 11 et dédié à ce module de création. A la suite d'une interaction de l'utilisateur, via l'écran 11, avec un composant de saisie, l'utilisateur peut, le cas échéant indiquer un nom de projet dédié à la création de cette nouvelle composition au moyen d'un composant de saisie, puis au moyen d'un composant graphique, par exemple un bouton de commande affiché sur l'écran 11, générer la création du nouveau projet.

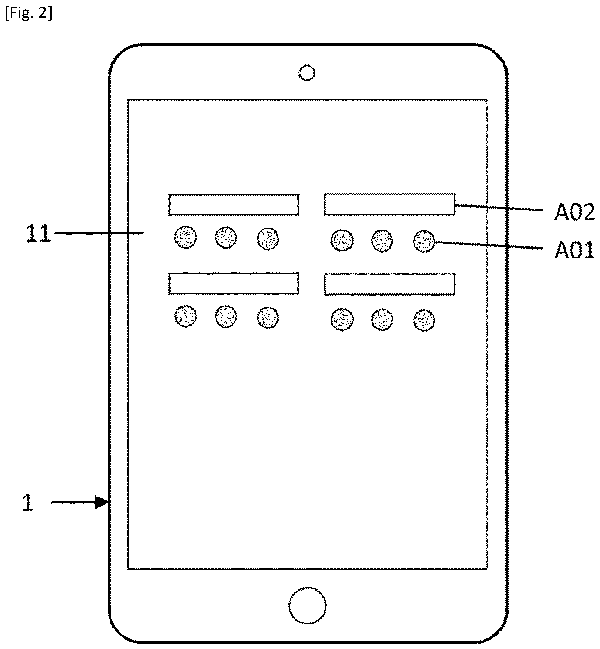

Dans une étape E11, à la suite de l'interaction de l'utilisateur, via l'écran 11, avec ce composant graphique de création de nouveau projet, l'application A contrôle l'écran 11 pour y afficher une interface de sélection d'un filtre F1 représentée en [Fig. 2], ladite interface de sélection comprenant un composant graphique A01 correspondant à un bloc comprenant au moins un filtre, ledit filtre représentant au moins une thématique santé. Dans l'exemple décrit, le composant A01 est un bouton circulaire permettant la sélection de la thématique santé d'intérêt.

Ainsi, lors de cette étape, l'utilisateur peut sélectionner une thématique santé parmi une pluralité de thématiques santé prédéterminées et stockées dans la base de données. Le cas échéant, plusieurs thématiques peuvent être regroupées pour former un ensemble homogène de thématiques santé, par exemple des thématiques santé liées au vieillissement ou encore associées à des troubles cognitifs. Dans l'exemple décrit, l'interface comprend également au moins un composant graphique d'affichage A02 dudit ensemble, ledit composant A02 peut comprendre information visuelle, par exemple un texte décrivant l'ensemble, par exemple un texte intitulé neuronutrition, nutricosmétique, ou encore nutrition associée au bien-être.

Cette interface de sélection d'une thématique santé peut également comporter plusieurs composants graphiques de sélection A01, chaque composant graphique de sélection A01 représentant une thématique santé parmi la pluralité de thématique prédéterminée, et présenter sous forme de pictogramme ou logo. Selon une variante, le composant graphique de sélection peut être sous forme d'une liste déroulante dont chaque item indique une thématique santé, par exemple choisi parmi une liste de thématique santé prédéterminée. L'application A pourra par exemple obtenir ladite liste de thématique par interrogation d'une base de données distante.

Selon une variante, l'étape E01 et l'étape E11 peuvent constituer une seule et unique étape. Dans ce cadre, le composant graphique permettant la création du nouveau projet est par exemple le composant A01 permettant simultanément la sélection d'un filtre représentant une thématique santé et la génération, création du nouveau projet.

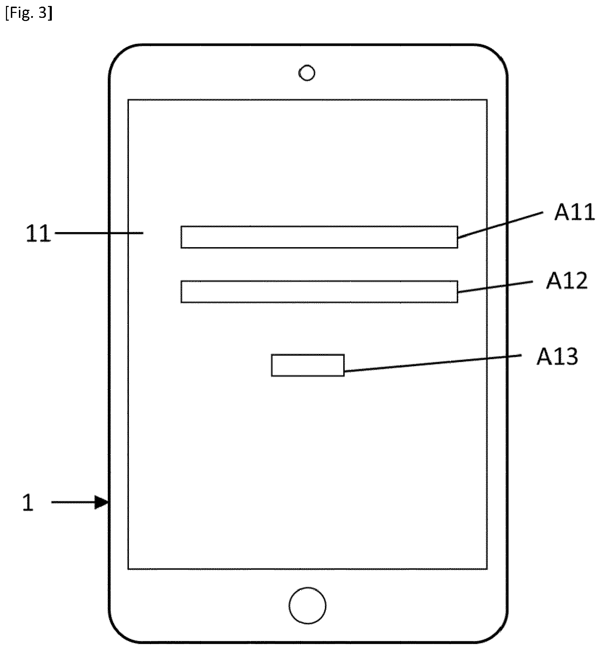

Dans une étape E12, à la suite d'une interaction de l'utilisateur, via l'écran 11, avec ce composant graphique de sélection A01 d'une thématique santé, l'application A contrôle l'écran 11 pour y afficher une nouvelle interface de sélection d'un filtre F2, représentée en [Fig. 3], ladite interface comprenant un deuxième bloc de filtre comprenant au moins un filtre dédié à la sélection d'au moins un territoire de commercialisation de la composition, d'une quantité ou d'un volume de composition souhaité par le client.

Cette nouvelle interface de sélection est dédiée, mais peut le cas échéant être associée à l'interface de sélection de la thématique santé. Cette interface comporte au moins un composant graphique de sélection du territoire A11 qui peut se présenter sous forme d'une liste déroulante dont chaque item indique un territoire de commercialisation prédéterminé ; au moins un composant graphique de sélection du volume A12 qui est un composant de saisie d'une réponse quantifiable, par exemple un nombre, via l'interface ; et au moins un composant graphique de validation A13, par exemple un bouton de confirmation des informations relatives aux territoires et au volume précédemment sélectionné.

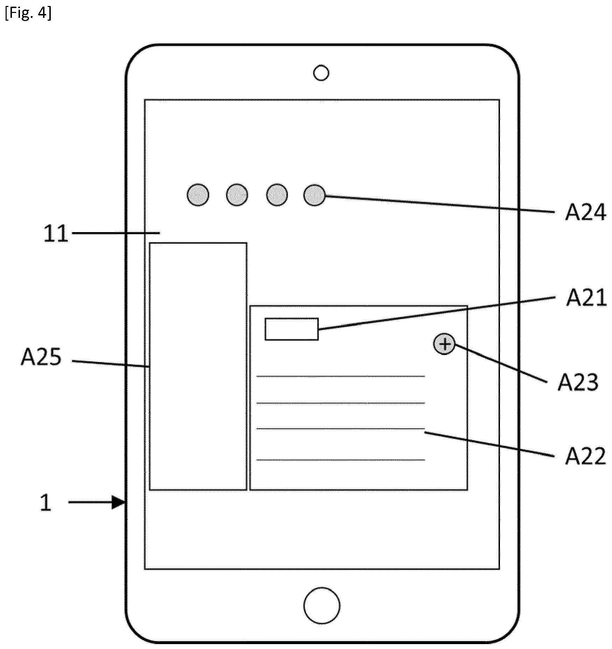

Dans une étape E21, à la suite d'une interaction de l'utilisateur, via l'écran 11, avec ce composant graphique de validation A13, l'application A contrôle l'écran 11 pour y afficher une nouvelle interface de sélection d'un ingrédient In parmi une pluralité d'ingrédients prédéterminés stockés dans une base de données, représentée en [Fig. 4]. Cette page comporte une pluralité de composants graphiques, dont au moins un composant graphique destiné à l'identification d'au moins un ingrédient A21, au moins un composant graphique destiné à la présentation dudit au moins un ingrédient A22 et au moins un composant graphique destiné à la sélection d'au moins un ingrédient A23 associé à une plage de doses de référence, au moins un composant graphique destiné à l'identification de la pluralité d'ingrédients prédéterminés A25. Cette page peut également comporter au moins un composant graphique A24 destiné à afficher un bloc comprenant une pluralité de filtres prédéterminés.

Aussi, la sélection, optionnelle, d'au moins un filtre, à l'aide du composant A24, permet d'afficher uniquement les ingrédients en fonction de ladite sélection. Par exemple la sélection d'un ingrédient naturel, biologique, végan, halal, ou kasher, mais également la sélection d'un ingrédient ayant une allégation de santé. L'utilisateur peut également s'informer des caractéristiques de l'ingrédient à l'aide du composant graphique A22, puis sélectionner l'ingrédient d'intérêt à l'aide du composant graphique A23.

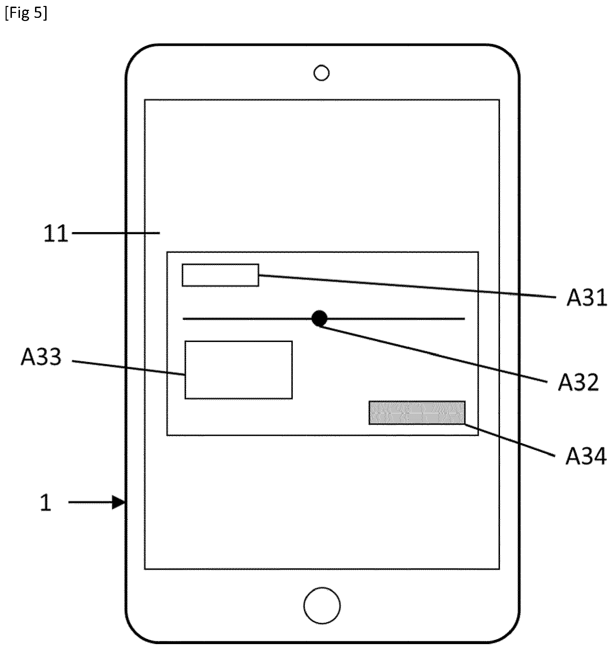

Dans une étape E31, à la suite d'une interaction de l'utilisateur, via l'écran 11, avec ce composant graphique de sélection A23, l'application A contrôle l'écran 11 pour y afficher une nouvelle interface de sélection d'une dose de l'ingrédient sélectionné, comprenant au moins un élément graphique de sélection manipulable A32 par l'utilisateur, représentée en [Fig. 5]. Cette interface comporte une pluralité de composants graphiques, dont au moins un composant graphique destiné à l'identification A31 de l'ingrédient sélectionné, le composant graphique manipulable destiné à la détermination d'une dose A32. A titre d'exemple, l'élément graphique de sélection manipulable A32 est un élément graphique de sélection déplaçable dans un plan cartésien selon un axe horizontale, par exemple un curseur déplaçable horizontalement sur un axe. Etant entendu que l'axe représente également la plage de doses de référence sélectionnée.

Dans une étape E32, à la suite d'une interaction de l'utilisateur, via l'écran 11, avec ce composant graphique de détermination d'une dose A32, l'application A contrôle l'écran 11 pour y afficher au moins une information visuelle A33 d'une dose de l'ingrédient sélectionné, et au moins un composant graphique A34 destiné à la sélection de l'ingrédient à la dose déterminée via le composant graphique A32. Par exemple, le composant graphique destiné à afficher l'information visuelle A33 peut être un logo et/ou un texte affiché via un changement de couleur, par exemple du gris au vert. L'utilisateur peut, ainsi déterminer la dose de l'ingrédient sélectionné à l'aide du composant A34 permettant l'affichage de l'information visuelle A33.

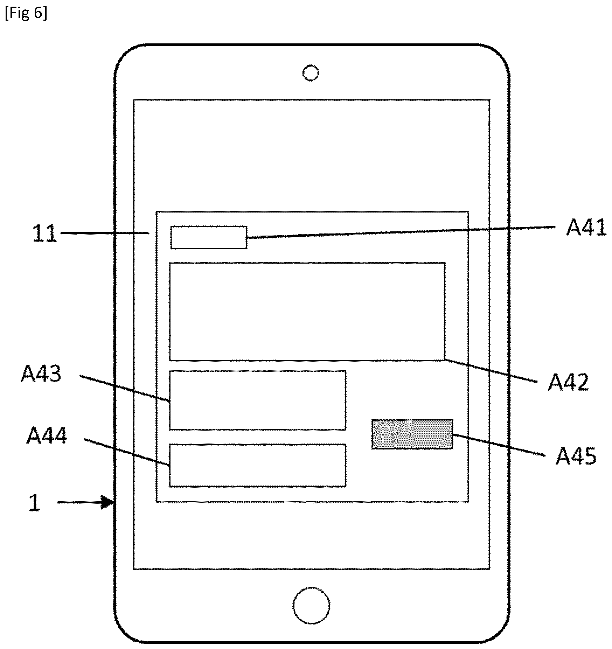

Dans une étape E41, à la suite d'une interaction de l'utilisateur, via l'écran 11, avec ce composant graphique de sélection A34, l'application A contrôle l'écran 11 pour y afficher une nouvelle interface de présentation de la composition, représentée en [Fig. 6]. Ladite interface de présentation comprenant un composant graphique d'identification de la composition A41, un composant graphique d'identification d'au moins un ingrédient présent dans ladite composition et de la dose sélectionnée A42, d'au moins un élément graphique de présentation des caractéristiques de la composition A43, et d'au moins un élément graphique de présentation d'une information économique A44, par exemple un prix indicatif de la composition. Par exemple, l'élément graphique de présentation des caractéristiques de la composition A43 peut comporter les informations suivantes, la présence d'une allégation de santé, et/ou d'une preuve scientifique. Cette page peut également comporter un élément graphique de validation d'une formulation ou d'une offre commercial A45, par exemple l'élément graphique A45 est un bouton de validation.

Selon une variante, lorsque l'élément graphique A45 est un élément graphique de validation d'une composition et à la suite d'une interaction de l'utilisateur, via l'écran 11, avec cet élément graphique A45, l'application A contrôle l'écran 11 pour y afficher une nouvelle interface de présentation d'au moins une composition validée. Ladite interface comprenant au moins un élément graphique de validation d'une offre commercial. A la suite d'une interaction de l'utilisateur, via l'écran 11, avec ce composant graphique, l'application A contrôle l'écran 11 pour générer directement un fichier comprenant l'offre, par exemple un fichier sous format PDF. Ledit fichier peut être envoyé directement par e-mail à au moins un destinataire, par exemple le client.

La description qui précède explique clairement comment l'invention permet d'atteindre les objectifs qu'elle s'est fixée, à savoir permettre à un utilisateur de recueillir les besoins d'un client, puis de générer une composition comprenant au moins un ingrédient actif à une dose déterminée selon différents critères prédéterminés, au cours d'un rendez-vous avec le client, dans un laps de temps réduit et de façon la plus fiable possible, grâce au terminal mobile apte à mettre en oeuvre le procédé selon l'invention, lui permettant ainsi d'avoir accès à de nombreuses connaissances et expertises, en particulier la réglementation en vigueur, lui permettant de déterminer la dose associée audit ingrédient.

En tout état de cause, l'invention ne saurait se limiter aux modes de réalisation spécifiquement décrits dans ce document, et s'étend en particulier à tous moyens équivalents et à toute combinaison techniquement opérante de ces moyens.

## Revendications

1. Procédé d'assistance dans la formulation d'une composition mis en oeuvre par un terminal mobile, dans lequel le procédé comprend les étapes suivantes :
a. Affichage sur l'écran du terminal mobile d'une interface de sélection, par un utilisateur du terminal mobile, d'un ingrédient parmi une pluralité d'ingrédients prédéterminés stockés dans une base de données ;
b. Sélection d'au moins une plage de doses de référence associée audit ingrédient sélectionné parmi une pluralité de doses de référence stockées dans une base de données,
c. Affichage sur l'écran du terminal mobile d'une interface de sélection d'une dose de l'ingrédient sélectionné, comprenant un élément graphique de sélection manipulable par un utilisateur du terminal mobile,
d. Détermination de la dose de l'ingrédient parmi une pluralité de doses prédéterminées dudit ingrédient, à partir de l'élément graphique de sélection,
e. Génération et affichage d'au moins une information visuelle sur l'écran du terminal, en fonction de la dose déterminée à l'étape précédente et de l'au moins une plage de doses de référence sélectionnée.

2. Procédé selon la revendication précédente, dans lequel la plage de doses de référence est sélectionnée en fonction d'au moins un critère choisi parmi la sélection d'une allégation de santé, d'une preuve d'efficacité, d'un territoire de commercialisation, d'une valeur de référence, et leur combinaison.

3. Procédé selon l'une des revendications précédentes, dans lequel l'élément graphique de sélection est déplaçable dans un plan cartésien selon un axe, un repère ou une position angulaire ; et/ou l'élément graphique de sélection est modifiable selon une forme et/ou une couleur; et/ou l'élément graphique de sélection est modifié lors de la sélection de la dose par l'utilisateur du terminal mobile.

4. Procédé selon l'une des revendications précédentes, dans lequel l'information visuelle est générée et affichée en fonction de la comparaison entre la dose déterminée et la plage de doses de référence.

5. Procédé selon l'une des revendications précédentes, dans lequel l'affichage est mis en oeuvre par le changement de couleur de l'information visuelle ou l'affichage de l'information visuelle elle-même.

6. Procédé selon l'une des revendications précédentes, dans lequel la plage de doses de référence est une pluralité de sous-plage de doses de référence.

7. Procédé selon l'une des revendications précédentes, dans lequel l'interface de sélection de l'au moins un ingrédient comporte au moins un premier bloc comprenant un filtre et ledit au moins un ingrédient est sélectionné parmi la pluralité d'ingrédients stockée dans la base de données, en fonction d'au moins une réponse audit bloc, préférentiellement le bloc de filtre comprend une interface de sélection d'une thématique santé, et/ou d'au moins un pays de commercialisation, et/ou d'un volume de composition.

8. Procédé selon la revendication précédente, dans lequel l'interface de sélection de l'au moins un ingrédient comporte un deuxième bloc comprenant un filtre et ledit au moins un ingrédient est sélectionné parmi la pluralité d'ingrédients stockée dans la base de données, en fonction d'au moins une réponse audit bloc, préférentiellement le bloc de filtre comprend une interface de sélection d'au moins une allégation de santé et/ou d'au moins une certification qualité.

9. Procédé selon l'une des revendications précédentes, dans lequel le procédé comprend également l'affichage sur l'écran du terminal mobile d'une interface de présentation de chaque ingrédient.

10. Procédé selon l'une des revendications précédentes, dans lequel le procédé comprend également une étape de sélection d'au moins un 2^{ème} ingrédient, dit ingrédient suggéré, parmi la pluralité d'ingrédients stockée dans la base de données en fonction de l'ingrédient sélectionné et de la dose dudit ingrédient déterminée et une étape d'affichage dudit ingrédient suggéré sur l'écran du terminal.

11. Procédé selon la revendication précédente, dans lequel la sélection de l'ingrédient suggérée est mise en oeuvre en fonction d'au moins un critère de compatibilité et/ou d'interaction entre les ingrédients sélectionnés, stocké dans une base de données.

12. Procédé selon l'une des revendications 7 à 11, dans lequel le procédé comprend également la génération et l'affichage d'au moins une information économique de la composition sur l'écran du terminal, en fonction du volume de composition sélectionné, et/ou la génération et l'affichage d'une interface de sélection, par un utilisateur du terminal mobile, d'au moins une option de service parmi une pluralité d'option de services prédéterminés stockés dans une base de données.

13. Procédé selon la revendication précédente, dans lequel le procédé comprend également l'affichage, au moyen d'une synchronisation instantanée, d'au moins une information économique modifiée de la composition, sur l'écran du terminal, en fonction de l'au moins une option de service sélectionnée.

14. Programme d'ordinateur comprenant un code de programme contenant des instructions qui, lorsqu'elles sont exécutées par un processeur, sont conçues pour mettre en oeuvre les étapes du procédé selon l'une des revendications précédentes.

15. Support de stockage de données sur lequel est enregistré le programme d'ordinateur selon la revendication 14.
